# EUROPÄISCHE PATENTSCHRIFT

(11) **EP 3 339 424 B1**
(45) Veröffentlichungstag und Bekanntmachung des Hinweises auf die Patenterteilung: **22.05.2019**
(21) Anmeldenummer: 16206714.4
(22) Anmeldetag: 23.12.2016
(51) Int. Cl.: C12M 1/42

(54) **MANUELLER INJEKTOR FÜR DIE ZELLMANIPULATION**
MANUAL INJECTOR FOR CELL MANIPULATION
INJECTEUR MANUEL POUR LA MANIPULATION DE CELLULES

(43) Veröffentlichungstag der Anmeldung: 27.06.2018
(73) Patentinhaber: Eppendorf AG, 22339 Hamburg (DE)
(72) Erfinder: Flucke, Christian, 22359 Hamburg (DE)
(74) Vertreter: Hauck Patentanwaltspartnerschaft mbB

(56) Entgegenhaltungen:
- Research Instruments: "SAS-SE Air Syringe", , 27. April 2010 (2010-04-27), XP002771875, Gefunden im Internet: URL:http://steptoemd.com/injector2.pdf [gefunden am 2017-07-10]
- Eppendorf: "CellTram Air CellTram Oil Cell tram Vario Operating manual", , 1. Januar 2009 (2009-01-01), XP002771876, Gefunden im Internet: URL:http://www.pocdscientific.com.au/files /POCDS_pdfs/CellTram_AirOilVario_operating _manual.pdf [gefunden am 2017-07-10]
- Narishige: "IM-11-2 Pneumatic Microinjector", , 1. Januar 1999 (1999-01-01), XP002771877, Gefunden im Internet: URL:http://products.narishige-group.com/gr oup1/IM-11-2/injection/english.html [gefunden am 2017-07-10]

## Beschreibung

Die Erfindung betrifft einen manuellen Injektor für die Zellmanipulation sowie eine Methode zur Zellmanipulation und die Verwendung des manuellen Injektors..

Typische Aufgaben bei der Zellmanipulation sind das Halten von Zellen, die Injektion von Lösungen, Zellen und Zellbestandteilen in Zellen oder Gewebe sowie die Entnahme oder Aufnahme von Zellen, Zellbestandteilen oder Lösungen. Hierfür werden Injektoren (Mikroinjektoren) verwendet. Diese umfassen eine Verdrängungsvorrichtung für ein Fluid, die über einen Schlauch mit einem Kapillarenhalter zum Halten einer austauschbaren Mikrokapillare verbunden ist. Ein manueller Injektor weist eine manuell angetriebene Verdrängungsvorrichtung auf. Eine Mikrokapillare ist eine Hohlnadel mit einer Spitze, die einen Innendurchmesser im Bereich von 1 bis 200 µm, vorzugsweise 4 bis 100 µm, aufweist. Vorzugsweise besteht die Mikrokapillare aus Glas.

Mittels der Verdrängungsvorrichtung werden in der Mikrokapillare Unterdrucke oder Überdrucke erzeugt, um Zellen, Zellbestandteile oder Lösungen mittels der Mikrokapillare zu halten, zu entnehmen oder zu injizieren. Für die Injektion kann eine Mikrokapillare in eine Zelle eingestochen werden. Hierbei kann die Zelle, welche im Zellmedium schwimmt, mittels einer weiteren Mikrokapillare gehalten werden. Ein Anwendungsbeispiel ist die In-Vitro-Fertilisation, bei der mittels einer Mikropapillare ein Spermium in eine mittels einer weiteren Mikrokapillare gehaltene Eizelle injiziert wird.

Bekannte Arbeitsplätze für die Zellmanipulation umfassen ein inverses Mikroskop zum Sichtbarmachen der Zellen und einen daran montierten, beweglichen Mikroskoptisch zum Platzieren der Zellen im Bildbereich. Am Mikroskop sind ein bis zwei Manipulatoren befestigt, die die Handbewegungen des Nutzers unter großer Untersetzung auf Mikrokapillaren als Werkzeug zur Manipulation der Zellen übertragen. Bei den bekannten Arbeitsplätzen sind sämtliche Komponenten sehr dicht nebeneinander auf einem schwingungsgedämpften Arbeitstisch angeordnet. Manuelle Injektoren sind jedoch relativ groß. Bedingt durch die Baugrößen und die Gestaltung und Anordnung der Bedienelemente sind bekannte manuelle Injektoren vergleichsweise bedienungsunfreundlich.

Bei pneumatischen Mikroinjektoren ist das Fluid Luft oder ein anderes Gas. Der pneumatische Mikroinjektor CellTram® Air der Eppendorf AG und der pneumatische Mikroinjektor IM11-2 der Narishige Group weisen in axialer Richtung hintereinander ein Einstellrad, eine in einer Spindelmutter verstellbare Gewindespindel und einen Kolben in einem Zylinder auf. Das Einstellrad befindet sich an einem Ende eines zylindrischen Gehäuses, welches Gewindespindel, Spindelmutter, Kolben und Zylinder enthält. Das Gehäuse ist im Wesentlichen horizontal ausgerichtet, wobei es an einem Gerätefuß geringfügig schwenkbar um eine horizontale Achse gelagert ist. Durch Verstellen des Einstellrades wird der Kolben im Zylinder verlagert und eine Luftsäule in der Mikrokapillare verschoben. Nachteilig ist, dass der Benutzer zum Verstellen des Einstellrades eine ergonomisch ungünstige Haltung annehmen muss, in der die Schulter hochgezogen, der Unterarm vom Tisch abgehoben und das Handgelenk angewinkelt ist. Falls er mit zwei Injektoren arbeitet, muss er die Haltung mit beiden Schultern und Armen annehmen.

Ferner ist die Leistungsfähigkeit pneumatischer Injektoren durch Kompressibilität der Luft begrenzt, bei schlechten Konstruktionen oder mangelhaften Geräten durch Leckagen. Durch Kompression oder Leckagen kann sich der Luftdruck im System allmählich verändern (Drift) und das Halten von Zellen sowie das Entnehmen und Injizieren von Material beeinträchtigt werden.

Zudem weisen die meisten pneumatischen Injektoren ein ungünstiges Verhältnis des Arbeitsvolumens der Kolben-Zylindereinheit und des Totvolumens im System auf, welches die Leistungsfähigkeit weiter einschränkt. Herkömmliche pneumatische Injektoren werden vom Benutzer vielfach als träge empfunden und sind nicht optimal für dynamische, d.h. verzögerungsarme auszuführende Manipulationsaufgaben, wie das Einfangen und Immobilisieren von Spermien oder Injizieren in Zellen. Insbesondere werden sie als träge empfunden bei in schneller Folge auszuführenden Wechseln zwischen verschiedenen Drücken, insbesondere bei Wechseln zwischen Überdrücken und/ oder Unterdrücken.

Falls die Gewindespindel ein steiles Gewinde aufweist, sind präzise Volumeneinstellungen schwierig. Weist sie ein feines Gewinde auf, ist eine Vielzahl von Umdrehungen des Einstellrades für eine Zellmanipulation erforderlich.

Der pneumatische Mikroinjektor IM11-2 der Narishige Group hat zwei koaxiale Gewindespindeln, von denen eine ein steiles Gewinde und die andere ein feines Gewinde aufweist, wodurch wahlweise sowohl ein zügiges Dosieren großer Volumina als auch ein feines Dosieren geringer Volumina ermöglicht wird. Nachteilig ist jedoch der hohe bauliche Aufwand, zumal ein zusätzliches, auf die steile Gewindespindel als Bremse wirkendes Reibelement aus Kunststoff verhindert, dass die komprimierte Luft im Zylinderraum den Kolben bewegt, da die Selbsthemmung der Gewindespindel-Spindelmutter-Paarung nicht ausreichend ist.

Die pneumatischen Mikroinjektoren SAS und SAS-SE der Research Instruments Ltd. haben ein säulenförmiges Drehrad, das über eine in einer Spindelmutter drehbaren Gewindespindel mit einem darunter angeordneten Kolben in einem Zylinder verbunden ist. Das Drehrad wird mit Daumen und Zeigefinger verstellt. Unterhalb des säulenförmigen Drehrades hat der Mikroinjektor einen scheibenförmigen Sockel. Aufgrund der hohen Übersetzung ist zwar eine schnelle Verstellung größerer Volumina möglich. Eine präzise Einstellung kleiner Volumina ist jedoch schwierig.

Bei hydraulischen Mikroinjektoren ist das Fluid eine Flüssigkeit. Der manuelle Mikroinjektor CellTram® Oil der Eppendorf AG und andere hydraulische Injektoren arbeiten mit einer Flüssigkeit als Verdrängungsfluid. Ein kleines Luftpolster in der Mikrokapillare verhindert einen direkten Kontakt zwischen der Flüssigkeit und dem zu behandelnden Material. Gegenüber pneumatischen Injektoren ist das Vermeiden des durch Kompressionseffekte bedingten Drift und das nahezu verzögerungsfreie Verschieben der Flüssigkeit von Vorteil. Nachteilig ist jedoch, dass der hydraulische Injektor blasenfrei mit Flüssigkeit befüllt werden muss und das Befüllen nach einer gewissen Anzahl von Manipulationen wiederholt werden muss. Außerdem besteht die Gefahr von Kontaminationen und Unverträglichkeiten durch Kontakt zwischen Flüssigkeit und Zellen.

Bei pneumatischen und hydraulischen Mikroinjektoren kann ein Spiel zwischen Gewindespindel und Spindelmutter eine Hysterese bewirken.

Davon ausgehend liegt der Erfindung die Aufgabe zugrunde, einen manuellen Injektor zum Verdrängen eines Fluids für die Zellmanipulation zur Verfügung zu stellen, der bedienungsfreundlicher ist, ohne Einsatz von Hydraulikmedium einen geringen Drift aufweist, ein dynamisches, d.h. verzögerungsarmes Arbeiten ermöglicht, ein Verdrängen großer Volumina mit geringem Einstellaufwand und ein genaues Einstellen kleiner Volumina ermöglicht.

Die Aufgabe wird durch einen manuellen Injektor mit den Merkmalen von Anspruch 1 gelöst. Vorteilhafte Ausgestaltungen des manuellen Injektors sind in Unteransprüchen angegeben.

Der erfindungsgemäße manuelle Injektor für die Zellmanipulation umfasst eine Verdrängungsvorrichtung zum Verdrängen eines Fluids, einen mit der Verdrängungsvorrichtung verbundenen Injektionsschlauch und einen mit dem Injektionsschlauch verbundenen Kapillarenhalter zum Halten einer Mikrokapillare oder eine mit dem Injektionsschlauch verbundene Mikrokapillare, wobei die Verdrängungsvorrichtung folgende Merkmale aufweist:
- einen kreisscheibenförmigen Grundkörper, der an einer ersten Stirnseite einen zu seiner Mittelachse konzentrischen, ringförmigen Zylinderraum aufweist,
- ein an der ersten Stirnseite angeordnetes, kreisscheibenförmiges, zum Grundkörper konzentrisches Drehrad,
- ein Schraubgetriebe umfassend eine Spindelmutter und eine darin eingreifende und auf der Mittelachse des Grundkörpers angeordnete Gewindespindel, wobei die Spindelmutter fest mit dem Drehrad und die Gewindespindel fest mit dem Grundkörper verbunden ist oder umgekehrt,
- einen im Zylinderrraum angeordneten, am Innenumfang an der Innenwand des Zylinderraumes und am Außenumfang an der Außenwand des Zylinderraumes abdichtend anliegenden, hohlzylindrischen Kolben,
- eine Drehentkopplung, die das Drehrad mit dem Kolben verbindet und ausgebildet ist, dass Kolben und Drehrad in Richtung der Mittelachse des Grundkörpers nicht relativ zueinander verlagerbar sind und um die Mittelachse herum relativ zueinander drehbar sind, und
- einen mit dem Zylinderraum verbundenen Anschluss, mit dem der Injektionsschlauch verbunden ist.

Die Verdrängungsvorrichtung des erfindungsgemäßen Injektors ist auf einen Arbeitstisch oder einen anderen Untergrund absetzbar, sodass der Grundkörper unten und das Drehrad oben angeordnet ist. Die Verdrängungsvorrichtung wird manuell durch Drehen des Drehrads relativ zum Grundkörper angetrieben. Die Drehung des Drehrades wird über die Spindelmutter und die Gewindespindel in eine Linearbewegung übersetzt, sodass sich das Drehrad in Richtung der Mittelachse des Grundkörpers näher zu diesem hin oder von diesem weg verlagert. Dabei nimmt das Drehrad mittels der Drehentkopplung den Kolben mit, der ein Fluid aus dem Zylinderraum herausdrückt oder in diesen hineinzieht. Dabei bewirkt die Drehentkopplung, dass das Drehrad den Kolben in Richtung der Mittelachse des Grundkörpers verlagert und nicht um die Mittelachse dreht. Vorzugsweise ist der Injektor ein pneumatischer Injektor und es handelt sich bei dem Fluid um Luft oder ein anderes Gas. Der Injektor kann aber auch ein hydraulischer Injektor und das Fluid eine Flüssigkeit sein. Durch die Verdrängung des Fluids kann über einen mit dem Anschluss verbundenen Injektionsschlauch Medium von einer Mikrokapillare angesaugt oder von dieser abgegeben werden.

Die Verdrängungsvorrichtung hat eine flache Bauweise, da das Schraubgetriebe, der Grundkörper mit dem Zylinderraum und das Drehrad mit dem Kolben koaxial ineinander angeordnet sind. Beim Bedienen kann der Arm des Benutzers in ergonomischer Position neben der Verdrängungsvorrichtung auf dem Tisch liegen. Dabei kann sich der Handrücken auf dem Tisch abstützen. Durch Kippen der Hand und Drehen des Drehrades mit den Fingern kann die Verdrängungsvorrichtung bedient werden. Bezogen auf die Abmessungen der Verdrängungsvorrichtung ist das durch Verlagerung des Kolbens im Zylinderraum verdrängbare Arbeitsvolumen vergleichsweise groß. Diese Eigenschaft resultiert aus den besonders großen Durchmessern der Grundfläche von Zylinderraum und Kolben. Ferner wird nur ein kleiner Hub des Kolbens benötigt, um eine große Volumenverdrängung zu erreichen. Bezogen auf eine Umdrehung des Drehrades kann ein verhältnismäßig großes Volumen verdrängt werden. Andererseits ist das verdrängte Volumen pro Bogenelement am Umfang des Drehrades verhältnismäßig gering, sodass auch eine sehr feinfühlige Bedienung möglich ist. Der Injektor weist im Vergleich zum Arbeitsvolumen (z.B. 20 ml) ein kleines Totvolumen (z.B. 0,5 ml, inkls. 1,3m Schlauch und Kapillarenhalter), sodass trotz der pneumatischen Wirkweise schnell hohe Drucke erreicht und gehalten werden können. Das Arbeitsvolumen ist der Rauminhalt, den der Kolben im Zylinder bei Maximalhub verdrängen kann. Das Totvolumen besteht aus Nebenräumen wie dem Innenvolumen des Kapillarenhalters, des Injektionsschlauchs oder Anschlüssen und kann nicht verdrängt werden. Arbeits- und Totvolumen stehen jedoch in keinem kausalen Zusammenhang, d.h. skalieren nicht miteinander. Bei der vorliegenden Erfindung gilt jedoch, dass das Totvolumen im Vergleich zum Arbeitsvolumen kleiner ist. Insbesondere ist das Totvolumen bei der erfindungsgemäßen Vorrichtung kleiner gleich als 1 ml.

Aufgrund des einfachen Aufbaus können die Herstellungskosten gering gehalten werden. Die Verdrängungsvorrichtung ist skalierbar, sodass beispielsweise kleine Ausführungen möglich sind, die nur zum Halten von Zellen dienen, und sich platzsparend an einem Arbeitsplatz für die Zellmanipulation unterbringen lassen (z.B. durch Anbringung, insbesondere Ankleben, an ein Mikroskop oder an ein Bedienpult eines Manipulators). Ferner können große Ausführungen verwirklicht werden, die insbesondere zum Injizieren von Materialien in Zellen benutzt werden können.

Die Verdrängungsvorrichtung kann besonders platzsparend ausgebildet werden, beispielsweise mit einer Höhe von maximal 5 cm, vorzugsweise im Bereich von 3 bis 4 cm, und mit einem Durchmesser von 30 bis 130 mm, vorzugsweise von 80 bis 100 mm für Injektoren für dynamische Anwendungen und vorzugsweise 40 bis 60 mm für Injektoren zum Halten von Zellen.

Gemäß einer bevorzugten Ausführungsart der Erfindung ist zwischen Grundkörper und Drehrad ein kombiniertes Linear- und Drehlager vorhanden, das ausgebildet ist, das Drehrad beim Verlagern relativ zum Grundkörper axial in Richtung der Mittelachse des Grundkörpers zu führen und radial um die Mittelachse des Grundkörpers herum zu lagern. Hierdurch kann eine besonders präzise Verlagerung des Kolbens im Zylinderraum erreicht werden. Grundsätzlich ist dies aber auch möglich, indem das Schraubgetriebe besonders präzise ausgebildet wird, z. B. durch toleranzarme Herstellung. Gemäß einer weiteren Ausführungsart ist hierfür das Schraubgetriebe ein Kugelgewindetrieb. Kugelgewindetriebe arbeiten konstruktionsbedingt besonders präzise.

Gemäß einer weiteren Ausführungsart weist das kombinierte Linear- und Drehlager eine Führungsbuchse und eine in der Führungsbuchse geführte Rundstange auf der Mittelachse des Grundkörpers auf, wobei die Führungsbuchse fest mit dem Grundkörper und die Rundstange fest mit dem Drehrad verbunden ist oder umgekehrt. Bei dieser Ausführungsart ist das kombinierte Linear- und Drehlager konstruktiv besonders einfach als radiales Gleitlager ausgebildet. Durch die Führungsbuchse und die darin geführte Rundstange auf der Mittelachse des Grundkörpers ist ein für eine präzise Führung besonders günstiges Verhältnis von Führungslänge und Führungsdurchmesser möglich.

Gemäß einer weiteren Ausführungsart ist die Führungsbuchse einteilig mit dem Grundkörper und/oder die Rundstange einteilig mit dem Drehrad ausgebildet oder umgekehrt. Durch die einteilige Ausbildung der Komponenten des Linear- und Drehlagers mit Grundkörper und Drehrad wird eine besonders präzise Führung des Drehrades bezüglich des Grundkörpers erreicht.

Gemäß einer weiteren Ausführungsart ist auf der Rundstange mindestens ein Gleitring angeordnet, der in der Führungsbuchse gleitet. Gleitring und Führungsbuchse können eine besonders reibungsarme Materialpaarung bilden. Hierdurch wird eine besonders leichtgängige und präzise einstellbare Drehung des Drehrades ermöglicht. Gemäß einer weiteren Ausführungsart ist auf der Rundstange eine zumindest teilweise umlaufende Ringnut und/oder ein mindestens teilweise umlaufender Vorsprung vorhanden, in der oder an dem der Gleitring auf der Rundstange gehalten ist.

Gemäß einer weiteren Ausführungsart ist die Spindelmutter einteilig mit dem Drehrad oder mit dem Grundkörper ausgebildet und/oder ist die Gewindespindel ein mittels eines Befestigungsmittels (z.B. Befestigungsschraube) am Grundkörper oder Drehrad befestigtes Bauteil.

Gemäß einer weiteren Ausführungsart ist die Rundstange zugleich als Spindelmutter ausgebildet, indem sie mit einem Innengewinde zum Einschrauben der Gewindespindel versehen ist. Hierdurch wird eine bauliche Vereinfachung erreicht.

Gemäß einer weiteren Ausführungsart ist die Drehentkopplung ein Drehlager, das ein erstes und ein zweites Drehlagerelement aufweist, die axial zueinander nicht verlagerbar und relativ zueinander drehbar sind, wobei das erste Drehlagerelement an dem Drehrad und das zweite Drehlagerelement an dem Kolben befestigt ist. Die Ausführung der Drehentkopplung als Drehlager ermöglicht baulich besonders einfache Lösungen. Gemäß einer weiteren Ausführungsart ist das Drehlager ein zum Grundkörper konzentrisches Radiallager, vorzugsweise ein radiales Wälzlager. Das Wälzlager ist beispielsweise ein Rillenkugellager. Es können einfache Standard-Wälzlager verwendet werden. Gemäß einer alternativen Ausführungsart ist das Drehlager ein Gleitlager.

Gemäß einer weiteren Ausführungsart besteht der Kolben aus einem oder mehreren der nachfolgenden Materialien: Metall oder Kunststoff, vorzugsweise Aluminium, oder ein anderes steifes, formhaltiges Material.

Gemäß einer weiteren Ausführungsart weist der Kolben auf der der ersten Stirnseite zugewandten Seite einen zur Mittelachse des Kolbens konzentrischen, hohlzylindrischen Ringraum auf, in dem die Drehentkopplung zumindest teilweise angeordnet ist. Hierdurch wird eine raumsparende Unterbringung der Drehentkopplung ermöglicht. Bei Ausführung der Drehentkopplung als Radiallager, insbesondere als radiales Wälzlager, kann der Ringraum vorteilhaft zur Unterbringung von Halteringen zum Fixieren von Innenring und Außenring des Wälzlagers dienen. Gemäß einer weiteren Ausführungsart bestehen die Halteringe aus einem oder mehreren der nachfolgenden Materialien: Metall oder Kunststoff, vorzugsweise rostfreier Stahl oder ein anderes steifes, formhaltiges Material.

Gemäß einer weiteren Ausführungsart weist der Kolben am Innenumfang und am Außenumfang jeweils mindestens einen umlaufenden ersten Kolbendichtring und mindestens einen umlaufenden zweiten Kolbendichtring auf, über die der Kolben gegenüber der Innenwand und der Außenwand des Zylinderraumes abgedichtet ist. Gemäß einer bevorzugten Ausführungsart sind die Kolbendichtringe aus einem elastischen Kunststoff gebildet. Hierdurch kann eine besonders leichtgängige und gute Abdichtung des Kolbens im Zylinderraum erreicht werden. Gemäß einer weiteren Ausführungsart besteht der Kolbendichtring aus einem oder mehreren der nachfolgenden Materialien: Elastischer Kunststoff, Naturkautschuk, vorzugsweise NBR (Nitrilkautschuk) oder vorzugsweise FKM (Fluorkautschuk).

Gemäß einer weiteren Ausführungsart weist das Drehrad am äußeren Rand einer kreisscheibenförmigen Deckwand einen umlaufenden, den Außenumfang des Grundkörpers zumindest teilweise überlappenden Mantel auf. Bei dieser Ausführungsart ist das Drehrad topfartig ausgebildet. Durch den Mantel wird der innere Aufbau der Verdrängungsvorrichtung geschützt.

Gemäß einer weiteren Ausführungsart weist zumindest ein Bauteil ausgewählt aus Drehrad, Gewindespindel und Grundkörper eine Positionsanzeige zum Anzeigen der Stellung des Kolbens im Zylinderraum auf. Gemäß einer weiteren Ausführungsart ist die Positionsanzeige eine Skala oder ein Zeiger. Beispielsweise ist die Positionsanzeige an einem Kopf der Gewindespindel angeordnet, der aus einem zentralen Loch des Drehrades nach oben vorsteht. Die Stellung des Kolbens ist am Rand des Loches auf der Positionsanzeige ablesbar, die vorzugsweise eine Skala ist. Alternativ ist die Positionsanzeige am Außenumfang des Grundkörpers angeordnet. Die Stellung des Kolbens ist an der Unterkante des Mantels auf der Positionsanzeige ablesbar, die vorzugsweise eine Skala ist.

Gemäß einer weiteren Ausführungsart ist der Zylinderraum mit einem Belüftungsventil verbunden, das eine Schließstellung aufweist, in der es den Zylinderraum von der Umgebung trennt, und eine Öffnungsstellung, in der es den Zylinderraum mit der Umgebung verbindet, sowie eine Betätigungseinrichtung, um das Belüftungsventil von der Schließstellung in die Öffnungsstellung und von der Öffnungsstellung in die Schließstellung zu bringen. Das Belüftungsventil kann dafür genutzt werden, einen Überdruck schnell abzubauen. Beispielsweise wird es eingesetzt, wenn infolge einer Verstopfung sich in der Mikrokapillare ein Überdruck aufbaut, der sich durch Lösen der Verstopfung plötzlich entspannen könnte, wodurch Luftblasen in das Zellmedium geblasen würden, so dass der Nutzer die Zellen nicht mehr sehen und damit nicht weiter arbeiten kann. Dies kann zu Probenverlust führen. Durch Schnellbelüftung kann eine Zerstörung kostbarer bzw. seltener Zellen vermieden werden.

Gemäß einer bevorzugten Ausführungsart ist die Betätigungseinrichtung so ausgebildet, dass durch manuelle Betätigung das Belüftungsventil von der Schließstellung in die Öffnungsstellung bringbar ist, wobei eine Rückstellfeder vorgespannt wird, sodass das Belüftungsventil nach Entlastung der Betätigungseinrichtung durch den Anwender selbsttätig in die Schließstellung zurückkehrt.

Gemäß einer weiteren Ausführungsart ist an einer zweiten Stirnseite des Grundkörpers, die von der ersten Stirnseite abgewandt ist, ein Gerätefuß angeordnet. Gemäß einer bevorzugten Ausführungsart ist der Gerätefuß so ausgebildet, dass er die Verdrängungsvorrichtung an einer Verlagerung aus einer Position hindert und/oder vor Erschütterungen schützt. Gemäß einer bevorzugten Ausführungsart ist der Gerätefuß eine massive Scheibe und/oder umfasst oder besteht aus mindestens einem Gummielement und/oder eine Antirutschmatte und/oder mindestens einem Magneten.

Ein weiterer Aspekt der vorliegenden Erfindung ist eine Methode zur Zellmanipulation unter Verwendung des erfindungsgemäßen Injektors gemäß einem der Ansprüche 16 bis 18. Der erfindungsgemäße Injektor eignet sich insbesondere für Methoden wie die *in-vitro* Fertilisation (IVF), die intrazytoplasmatischen Spermieninjektion (ISCI), den Transfer von Mitochondrien und / oder den Zellkerntransfer. Diese Methoden umfassen gemäß einer weiteren Ausführungsart folgende Schritte: (a) das Anschließen einer Mikrokapillare an den erfindungsgemäßen Injektor; (b) die Auswahl einer zu manipulierenden Zelle; (c) die Annäherung der an den erfindungsgemäßen Injektor angeschlossen Mikrokapillare an die ausgewählte Zelle; (d) bei Berührung der Oberfläche (je nach Zelltyp Zellwand und oder Zellmembran) der Zelle durch die Mikrokapillare, Anlegen eines Unterdrucks vermittelt durch den Injektor, zum Halten der Zelle.

Für den eigentlichen Injektionsvorgang, wird eine mit Injektionsmaterial gefüllte Kapillare an die Zelloberfläche (je nach Zelltyp Zellwand und oder Zellmembran) einer Zelle angenähert, durch die Zelloberfläche hindurchgeschoben so dass die Kapillare in das Cytoplasma eindringt (penetrierende Berührung). Je nach Anwendung, wird die Kapillare noch genauer positioniert, z.B. in den sich im Cytoplasma befindlichen Pronukleus oder den Nukleus selber. Vermittelt durch den erfindungsgemäßen Injektor wird nun ein Überdruck angelegt, um das Injektionsmaterial, insbesondere eine Lösung, eine Suspension (z.B. eine Zellsuspension, eine Nukleinsäuresuspension etc.), eine Zelle (insbesondere ein Spermium), Zellmaterial, insbesondere Cytoplasma, Cytoplasmabestandteile, wie Mitochondrien und /oder Pronukleus, in das Cytoplasma (der Begriff Cytoplasma umfasst auch besondere Bereiche des Cytoplasmas, wie z.B. die unmittelbare Umgebung des Nukleus, zu injizieren.

Für einen Entnahmevorgang wird die Kapillare an die Zelloberfläche (je nach Zelltyp Zellwand und oder Zellmembran) einer Zelle angenähert, durch die Zelloberfläche hindurchgeschoben so dass die Kapillare in das Cytoplasma eindringt (penetrierende Berührung). Vermittelt durch den erfindungsgemäßen Injektor wird nun ein Unterdruck angelegt, um vermittelt durch den Injektor einen Zellbestandteil zu entnehmen, insbesondere den Zellkern, den Pronukleus, Mitochondrien, und/oder andere Zellbestandteile. Diese aufgeführten Methoden finden üblicherweise Verwendung in einem medizinischen, biotechnologischen, biochemischen, zellbiologischen und/oder forensischen Labor.

Die Erfindung wird nachfolgend anhand der anliegenden Zeichnungen eines Ausführungsbeispiels näher erläutert. In den Zeichnungen zeigen:

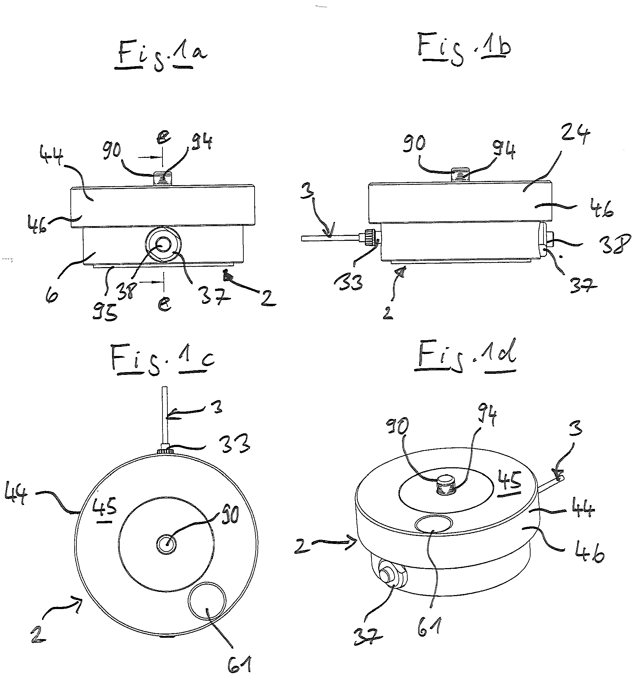

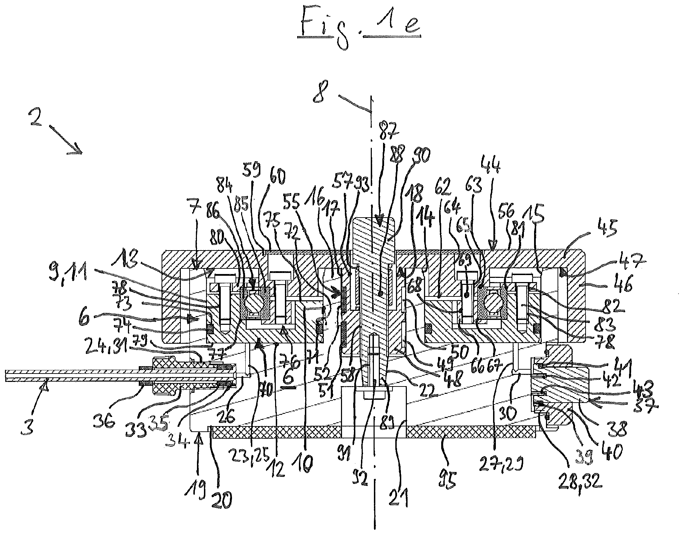

- Fig. 1a-e: Manueller Injektor in Vorderansicht (Fig. la), Seitenansicht von der rechten Seite (Fig. 1b), Draufsicht (Fig. 1c), in einer Perspektivansicht von oben und von der Seite (Fig. 1d) und in einem Schnitt entlang der Linie e-e von Fig. la (Fig. le);

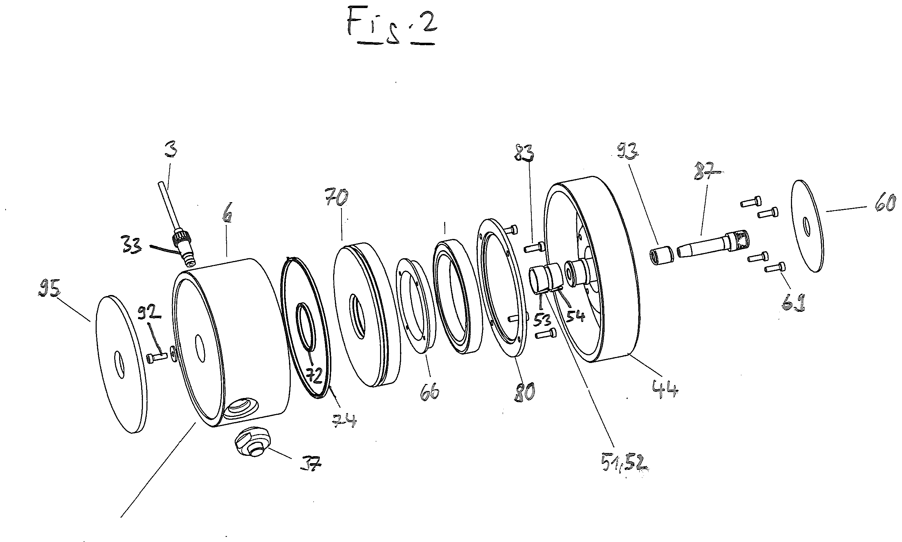

- Fig. 2: derselbe Injektor in einem perspektivischen Sprengbild.

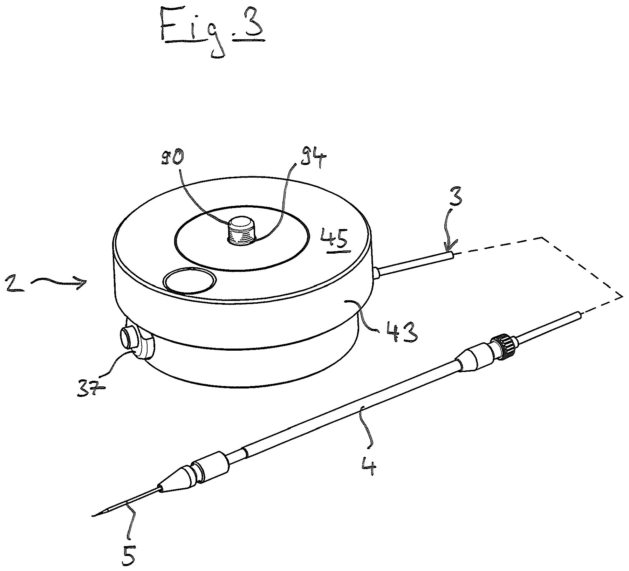

- Fig.3: derselbe Injektor mit Injektionsschlauch zusammen mit Kapillarenhalter und einer Mikrokapillare.

In der vorliegenden Anmeldung beziehen sich die Angaben "vertikal" und "horizontal", "oben" und "unten" und davon abgeleitete Angaben wie "darüber" und "darunter", "Oberseite und "Unterseite" auf eine Ausrichtung der Verdrängungsvorrichtung, bei der die Mittelachse von Grundkörper und Drehrad vertikal ausgerichtet und das Drehrad zumindest teilweise oberhalb des Grundkörpers angeordnet ist.

Der Injektor 1 weist eine Verdrängungsvorrichtung 2, einen Injektionsschlauch 3 und einen Kapillarenhalter 4 auf, in dem eine Mikrokapillare 5 gehalten ist (hierzu fehlt noch die Zeichnung).

Die Verdrängungsvorrichtung 2 umfasst einen kreisscheibenförmigen Grundkörper 6, der an einer ersten Stirnseite 7 einen zu seiner Mittelachse 8 konzentrischen, hohlzylindrischen Zylinderraum 9 aufweist. Der Zylinderraum 9 hat eine zylindrische Innenwand 10 und eine zylindrische Außenwand 11 sowie eine kreisringscheibenförmige Bodenwand 12.

Der Zylinderraum 9 hat an der ersten Stirnseite 7 eine kreisringförmige Zylinderöffnung 13, durch die hindurch er von der ersten Stirnseite 7 aus zugänglich ist. An der Zylinderöffnung 13 weist sowohl die Innenwand 10 als auch die Außenwand 11 des Zylinderraumes 9 eine Fase 14, 15 auf.

Ferner ist an der ersten Stirnseite 7 des Grundkörpers 6 eine Führungsbuchse 16 vorhanden, deren Mittelachse mit der Mittelachse 8 des Grundkörpers 6 zusammenfällt. Die Führungsbuchse 16 ist einteilig mit dem Grundkörper 6 ausgebildet, beispielsweise durch Einbringen einer zentralen ersten Sackbohrung 17.

Die Führungsbuchse 16 hat an der ersten Stirnseite 7 eine Buchsenöffnung 18, durch die die hindurch Führungsbuchse 16 von einer ersten Stirnseite 7 aus zugänglich ist.

Der Grundkörper 6 hat an seiner zweiten Stirnseite 19 eine kreisscheibenförmige erste Vertiefung 20. Ferner weist der Grundkörper 6 an der zweiten Stirnseite 19 eine zentrale zweite Sackbohrung 21 auf, deren Mittelachse mit der Mittelachse 8 Grundkörpers 6 zusammenfällt. Die zweite Sackbohrung 21 hat an der zweiten Stirnseite 19 eine Öffnung, durch die hindurch sie von der zweiten Stirnseite 19 aus zugänglich ist.

Ausgehend vom Boden der Führungsbuchse 16 erstreckt sich zum Boden der zweiten Sackbohrung 21 eine konische Bohrung 22, die sich von der Führungsbuchse 16 zur zweiten Sackbohrung 21 hin verjüngt.

Ein erster Verbindungskanal 23 verbindet den Zylinderraum 9 mit einer dritten Sackbohrung 24, die sich vom Außenumfang des Grundkörpers 6 radial in diesen hinein erstreckt. Der erste Verbindungskanal 23 ist durch zwei einander schneidende Kanalbohrungen 25, 26 gebildet, von denen eine ausgehend von der Bodenwand 12 des Zylinderraums 9 aus sich vertikal in den Grundkörper hinein erstreckt und die andere vom Boden der dritten Sackbohrung 24 aus sich radial in den Grundkörper 6 hinein erstreckt.

Diametral gegenüber dem ersten Verbindungskanal 23 ist der Zylinderraum 9 über einen zweiten Verbindungskanal 27 mit einer vierten Sackbohrung 28 verbunden, die sich vom Umfang des Grundkörpers 6 aus radial in diesen hinein erstreckt. Der zweite Verbindungskanal 27 ist ebenfalls durch zwei einander schneidende Kanalbohrungen 29, 30 gebildet. Die dritte und vierte Sackbohrung 24, 28 sind als Gewindebohrungen jeweils mit einem Innengewinde 31, 32 ausgebildet.

Ein druckdichter Schlauchanschluss ist wie folgt ausgeführt: in die dritte Sackbohrung 24 ist ein Schlauchfitting 33 eingeschraubt, um den Injektionsschlauch 3 anzuschließen.

Das Schlauchfitting 33 ist als Gewindehülse ausgebildet. Das Schlauchfitting 33 dichtet den Injektionsschlauch 3 mittels eines Schlauchflansches 34, der direkt aus dem Schlauchmaterial des Injektionsschlauches 3 geformt ist, gegen den Grund der Sackbohrung 24 ab. Das Schlauchfitting 33 ist axial beweglich auf den Injektionsschlauch 3 aufgefädelt und verspannt den Schlauchflansch 34 mittels des Gewindes der Sackbohrung 24 gegen den Grund der Sackbohrung 24. Eine erste Hülse 35 sorgt für den richtigen Abstand und ein O-Ring am Umfang der ersten Hülse 35 wird hauptsächlich als Federelement genutzt, weist aber auch eine zusätzliche Dichtwirkung auf.

Auf dem Injektionsschlauch 3 sitzt eine zweite Hülse 36, die sich an der äußeren Stirnseite des Schlauchfittings 33 abstützt und diesen vor dem Einschrauben in den Grundkörper 6 an einem Verrutschen auf dem Injektionsschlauch 3 hindert.

In der vierten Sackbohrung 28 ist ein Belüftungsventil 37 angeordnet. Dieses weist einen Ventilknopf 38 mit einem Flansch 39 am inneren Ende auf, der mittels einer in das Innengewinde 32 der vierten Sackbohrung 28 eingeschraubten Überwurfmutter 40 in der vierten Sackbohrung 28 gehalten ist. In der gezeigten Ausgangsstellung liegt der Flansch 39 abdichtend an der inneren Stirnseite 41 der Überwurfmutter 40 an. Der Ventilknopf 38 hat an seiner inneren Stirnseite einen umlaufenden zentralen Einstich 42, in den eine erste Schraubenfeder 43 eingesetzt ist, die sich am Boden der vierten Sackbohrung 28 abstützt. Ventilknopf 38 und Schraubenfeder 43 sind eine Betätigungseinrichtung.

Beim Eindrücken des Ventilknopfes 38 wird die Schraubenfeder 43 etwas zusammengedrückt und ein Belüftungsspalt zwischen Ventilknopf 38 und Überwurfmutter 40 geöffnet. Bei Entlastung des Ventilknopfes 38 drückt die Schraubenfeder 43 den Ventilknopf 38 zurück in die Ausgangsstellung.

Der Grundkörper 6 ist beispielsweise einteilig aus Aluminium oder einem anderen Metall oder aus Kunststoff hergestellt. Vorzugsweise ist er massiv ausgebildet.

Ferner weist die Verdrängungsvorrichtung 2 ein Drehrad 44 mit einer kreisscheibenförmigen Deckwand 45 auf. Um den äußeren Rand der Deckwand 45 läuft ein Mantel 46 um, sodass das Drehrad 44 insgesamt topfförmig ist.

Auf der zweiten Stirnseite 47 der Deckwand 45, die der ersten Stirnseite 7 des Grundkörpers 6 zugewandt ist, steht zentral eine Rundstange 48 vor. Die Rundstange 48 hat am Außenumfang zwei umlaufende Ringnuten 49, 50. In jede Ringnut 49, 50 ist ein Gleitring 51, 52 eingesetzt. Jeder Gleitring 51, 52 hat einen in Längsrichtung verlaufenden Schlitz 53, 54, der ein Aufweiten des Gleitringes 51, 52 ermöglicht, um diesen auf die Rundstange 48 aufzuschieben und in eine Ringnut 49, 50 einzusetzen. Die Gleitringe 51, 52 stehen etwas über den Außenumfang der Rundstange 48 über und liegen am Innenumfang der Führungsbuchse 16 an. Gemeinsam mit der Führungsbuchse 16 bildet die Rundstange 48 ein kombiniertes Dreh- und Gleitlager 55.

Das Drehrad 44 hat an seiner ersten Stirnseite 56 eine zentrale fünfte Sackbohrung 57. Die Rundstange 48 ist mit einer Gewindebohrung 58 versehen, die sich in der Rundstange 48 vom Boden der zentralen fünften Sackbohrung 57 bis zur zweiten Stirnseite 47 erstreckt. Die Gewindebohrung 58 ist als Spindelgewinde zur Aufnahme einer Gewindespindel ausgebildet.

Die erste Stirnseite 56 des Drehrades 44 weist eine kreisscheibenförmige zweite Vertiefung 59 auf. In der zweiten Vertiefung 59 ist eine Abdeckscheibe 60 angeordnet. Diese besteht beispielsweise aus Kunststoff, Gummi oder Metall. Sie kann insbesondere eine Skala oder Markierung tragen oder aus einem hautfreundlichen Material gebildet sein.

In der ersten Stirnseite 56 hat das Drehrad 44 eine Fingermulde 61.

Auf ihrer zweiten Stirnseite 47 hat die Deckwand 45 des Drehrades 44 einen zur Rundstange 48 konzentrischen, kreisscheibenförmigen Sockel 62. Der Sockel 62 hat zwischen Außenumfang und Unterseite eine umlaufende erste Schulter 63. Von der ersten Stirnseite 56 der Deckwand 45 erstrecken sich parallel zur Rundstange 48 sechste Sackbohrungen 64 in das Drehrad 44 hinein. Vom Boden der sechsten Sackbohrungen64 ausgehende Durchgangslöcher 65 erstrecken sich bis zur Unterseite des Sockels 62.

Das Drehrad 44 ist beispielsweise aus Aluminium oder einem anderen Metall oder aus Kunststoff hergestellt. Das Drehrad 44 ist vorzugsweise einteilig hergestellt.

Unterhalb des Sockels 62 ist ein unterer Haltering 66 angeordnet, der am Außenumfang eine zweite Schulter 67 aufweist. Der untere Haltering 66 weist erste Gewindebohrungen 68 auf. Er ist mittels erster Befestigungsschrauben 69 am Drehrad 44 gehalten, welche in die sechsten Sackbohrungen 64 eingesetzt sind und die Durchgangslöcher 65 durchgreifen, in die ersten Gewindebohrungen 68 eingeschraubt sind und sich mit ihren Schraubenköpfen am Boden der sechsten Sackbohrungen 64 abstützen.

Im Zylinderraum 9 ist ein Kolben 70 angeordnet, der im Wesentlichen die Form einer Kreisringscheibe aufweist. Der Kolben 70 hat am Innenumfang eine umlaufende erste Kolbenringnut 71, in der ein erster elastischer Kolbendichtring 72 sitzt. Am Außenumfang hat er eine zweite umlaufende Kolbenringnut 73, in der ein elastischer zweiter Kolbendichtring 74 sitzt. Der erste Kolbendichtring 72 dichtet den Kolben 70 an der Innenwand des Zylinderraums 9 und der zweite Kolbendichtring 74 dichtet den Kolben 70 an der Außenwand des Zylinderraums 9 ab.

Der Kolben 70 hat an seiner ersten Stirnseite 75, die der Zylinderöffnung 13 des Zylinderraumes 9 zugewandt ist, einen zur Mittelachse des Kolbens 70 konzentrischen, hohlzylindrischen Ringraum 76. Der Ringraum 76 hat in der Außenwand eine dritte Schulter 77. Ferner weist er an seiner ersten Stirnseite 75 zweite Gewindebohrungen 78 auf, die sich parallel zu seiner Mittelachse erstrecken. Die zweite Stirnseite 79 des Kolbens 70 ist glatt und bildet den Kolbenboden.

Der Kolben 70 ist beispielsweise aus Kunststoff oder aus Metall hergestellt.

Oberhalb des Kolbens 70 ist ein kreisringscheibenförmiger, oberer Haltering 80 angeordnet, der am Innenumfang eine vierte Schulter 81 aufweist. Der obere Haltering 80 hat Durchgangslöcher 82, die sich parallel zur Mittelachse des Kolbens 70 erstrecken. Durch die Durchgangslöcher 82 sind zweite Befestigungsschrauben 83 hindurchgesteckt und in die zweiten Gewindebohrungen 78 des Kolbens 70 eingeschraubt, bis die Köpfe der zweiten Befestigungsschrauben 83 an der Oberseite des oberen Halteringes 80 anliegen.

Ferner umfasst die Verdrängungsvorrichtung 2 ein Wälzlager 84, das als Rillenkugellager ausgebildet ist. Das Wälzlager 84 ist zwischen Drehrad 44 und Kolben 70 angeordnet. Der Innenring 85 des Wälzlagers 84 stützt sich oben an der ersten Schulter 63 am Sockel 62 des Drehrades 44 und unten an der zweiten Schulter 67 des unteren Halteringes 66 ab.

Der Innenring 85 ist durch Anziehen des unteren Halteringes 66 mittels der ersten Befestigungsschrauben 69 festgespannt.

Der Außenring 86 des Wälzlagers 84 stützt sich zwischen der dritten Schulter 77 des Kolbens 70 und der vierten Schulter 81 des oberen Halteringes 80 ab und ist durch Festziehen der zweiten Befestigungsschrauben 83 zwischen oberem Haltering 80 und Kolben 70 festgespannt.

Das Wälzlager 84 hält den Kolben 70 in einer definierten axialen Lage bezüglich des Drehrades 44 fest und ermöglicht ein Drehen des Drehrades 44 relativ zum Kolben 70. Hierdurch wird eine Drehentkopplung zwischen Drehrad 44 und Kolben 70 gebildet.

Ferner umfasst die Verdrängungsvorrichtung 2 eine Gewindespindel 87. Diese weist einen Spindelschaft 88 mit einem Konus 89 am unteren Ende und einem Spindelkopf 90 am oberen Ende auf. In der unteren Stirnseite der Gewindespindel 87 ist auf der Mittelachse der Gewindespindel 87 eine dritte Gewindebohrung 91 angeordnet.

Die Gewindespindel 87 ist beispielsweise aus Kunststoff oder Metall hergestellt.

Die Gewindespindel 87 ist in die fünfte Sackbohrung 57 des Drehrades 44 eingesetzt und in die Gewindebohrung 58 der Rundstange 48 eingeschraubt. Der Konus 89 ist in die konische Bohrung 22 des Grundkörpers 6 eingesetzt. Mittels einer von unten in die zweite Sackbohrung 21 des Grundkörpers 6 eingeführten dritten Befestigungsschraube 92 ist die Gewindespindel 87 am Grundkörper 6 fixiert. Zwischen dem Spindelkopf 90 und dem Boden der fünften Sackbohrung 57 des Drehrades 44 ist eine zweite Schraubenfeder 93 am Umfang der Gewindespindel 87 geführt. Die zweite Schraubenfeder 93 dient dazu, das Spiel zwischen Gewindespindel 87 und Gewindebohrung 58 aufzuheben.

Der Spindelkopf 90 hat an der Außenseite eine Positionsanzeige 94 mit einer Skala.

In der ersten Vertiefung 20 ist ein kreisringscheibenförmiger Gerätefuß 95 angeordnet, der beispielsweise als Gummischeibe, Magnetscheibe oder als Antirutschmatte (*sticky pad*) ausgebildet ist.

Das andere Ende des Injektionsschlauchs 3 ist mit dem Kapillarenhalter 4 verbunden. Der Kapillarenhalter 4 ist rohrförmig und ausgebildet, die auswechselbare Mikrokapillare 5 einzuklemmen.

Beim Drehen des Drehrades 44 führt seine Gewindebohrung 58 auf der Gewindespindel 87 eine Schraubbewegung aus. Hierbei wird das Drehrad 44 durch die Lagerung der Rundstange 48 in der Führungsbuchse 16 in Längsrichtung und Radialrichtung präzise bezüglich des Grundkörpers 6 geführt. Da die Gewindespindel 87 am Grundkörper 6 fixiert ist, wird das Drehrad 44 axial bezüglich des Grundkörpers 6 verlagert. Je nach Drehrichtung des Drehrades 44 bewegt es sich axial nach unten oder nach oben. Durch die Verbindung des Drehrades 44 über das Wälzlager 84 mit dem Kolben 70 wird der Kolben 70 in Axialrichtung mit verlagert, aber nicht mitgedreht, sodass zwischen Drehrad 44 und Kolben 70 eine Drehentkopplung vorhanden ist.

Durch die Verlagerung des Kolbens 70 wird Luft im Zylinderraum 9 verdrängt und eine Luftsäule im Injektionsschlauch 3 verschoben. Durch Verschiebung der Luftsäule kann Material aus der Mikrokapillaren 5 ausgestoßen oder in diese hereingesogen oder gehalten werden.

### Bezugszeichenliste

- 1: Injektor
- 2: Verdrängungsvorrichtung
- 3: Injektionsschlauch
- 4: Kapillarenhalter
- 5: Mikrokapillare
- 6: Grundkörper
- 7: erste Stirnseite
- 8: Mittelachse
- 9: Zylinderraum
- 10: Innenwand
- 11: Außenwand
- 12: Bodenwand
- 13: Zylinderöffnung
- 14, 15: Fase
- 16: Führungsbuchse
- 17: erste Sackbohrung
- 18: Buchsenöffnung
- 19: zweite Stirnseite
- 20: erste Vertiefung
- 21: zweite Sackbohrung
- 22: konische Bohrung
- 23: erster Verbindungskanal
- 24: dritte Sackbohrung
- 25, 26: Kanalbohrungen
- 27: zweiter Verbindungskanal
- 28: vierte Sackbohrung
- 29, 30: Kanalbohrungen
- 31, 32: Innengewinde
- 33: Schlauchfitting
- 34: Schlauchflansch
- 35: erste Hülse
- 36: zweite Hülse
- 37: Belüftungsventil
- 38: Ventilknopf
- 39: Flansch
- 40: Überwurfmutter
- 41: Stirnseite
- 42: Einstich
- 43: Schraubenfeder
- 44: Drehrad
- 45: Deckwand
- 46: Mantel
- 47: Stirnseite
- 48: Rundstange
- 49, 50: Ringnuten
- 51, 52: Gleitring
- 53, 54: Schlitz
- 55: kombiniertes Dreh- und Gleitlager
- 56: Stirnseite
- 57: fünfte Sackbohrung
- 58: Gewindebohrung
- 59: Vertiefung
- 60: Abdeckscheibe
- 61: Fingermulde
- 62: Sockel
- 63: erste Schulter
- 64: sechste Sackbohrung
- 65: Durchgangslöcher
- 66: unterer Haltering
- 67: zweite Schulter
- 68: Gewindebohrungen
- 69: erste Befestigungsschrauben
- 70: Kolben
- 71: erste umlaufende Kolbenringnut
- 72: erster Kolbendichtring
- 73: zweite umlaufende Kolbenringnut
- 74: zweiter Kolbendichtring
- 75: Stirnseite
- 76: Ringraum
- 77: dritte Schulter
- 78: zweite Gewindebohrungen
- 79: zweite Stirnseite
- 80: oberer Haltering
- 81: vierte Schulter
- 82: Durchgangslöcher
- 83: zweite Befestigungsschrauben
- 84: Wälzlager
- 85: Innenring
- 86: Außenring
- 87: Gewindespindel
- 88: Spindelschaft
- 89: Konus
- 90: Spindelkopf
- 91: dritte Gewindebohrung
- 92: dritte Befestigungsschraube
- 93: zweite Schraubenfeder
- 94: Positionsanzeige
- 95: Gerätefuß

## Patentansprüche

1. Manueller Injektor für die Zellmanipulation umfassend eine Verdrängungsvorrichtung (2) zum Verdrängen eines Fluids, einen mit der Verdrängungsvorrichtung (2) verbundenen Injektionsschlauch (3) und einen mit dem Injektionsschlauch (3) verbundenen Kapillarenhalter (4) zum Halten einer Mikrokapillare (5) oder einer mit dem Injektionsschlauch (3) verbundenen Mikrokapillare (5), wobei die Verdrängungsvorrichtung (2) folgende Merkmale aufweist:
• einen kreisscheibenförmigen Grundkörper (6), der an einer ersten Stirnseite (7) einen zu seiner Mittelachse (8) konzentrischen, hohlzylindrischen Zylinderraum (9) aufweist,
• ein an der ersten Stirnseite (7) angeordnetes, kreisscheibenförmiges, zum Grundkörper (6) konzentrisches Drehrad (44),
• ein Schraubgetriebe umfassend eine Spindelmutter und eine darin eingreifende und auf der Mittelachse (8) des Grundkörpers (6) angeordnete Gewindespindel (87), wobei die Spindelmutter fest mit dem Drehrad (44) und die Gewindespindel (87) fest mit dem Grundkörper (6) verbunden oder umgekehrt ist,
• einen im Zylinderraum (9) angeordneten, am Innenumfang an der Innenwand (10) des Zylinderraumes (9) und am Außenumfang an der Außenwand (11) des Zylinderraumes (9) abdichtend anliegenden, hohlzylindrischen Kolben (70),
• eine Drehentkopplung, die das Drehrad (44) mit dem Kolben (70) verbindet und ausgebildet ist, dass Kolben (70) und Drehrad (44) in Richtung der Mittelachse (8) des Grundkörpers (6) nicht relativ zueinander verlagerbar sind und um die Mittelachse (8) herum relativ zueinander drehbar sind, und
• einen mit dem Zylinderraum (9) verbundenen Anschluss (33), mit dem der Injektionsschlauch (3) verbunden ist.

2. Injektor nach Anspruch 1, bei dem zwischen Grundkörper (6) und Drehrad (44) ein kombiniertes Linear- und Drehlager (55) vorhanden ist, das ausgebildet ist, das Drehrad (44) beim Verlagern relativ zum Grundkörper (6) axial in Richtung der Mittelachse (8) des Grundkörpers (6) zu führen und radial um die Mittelachse (8) des Grundkörpers (6) herum zu lagern.

3. Injektor nach Anspruch 1 oder 2, bei dem das kombinierte Linear- und Drehlager (55) eine hohlzylindrische Führungsbuchse (16) und eine in der Führungsbuchse (16) geführte Rundstange (48) auf der Mittelachse (8) des Grundkörpers (6) aufweist, wobei die Führungsbuchse (16) fest mit dem Grundkörper (6) und die Rundstange (48) fest mit dem Drehrad (44) verbunden ist oder umgekehrt.

4. Injektor nach Anspruch 3, bei dem die Führungsbuchse (16) einteilig mit dem Grundkörper (6) und/oder die Rundstange (48) einteilig mit dem Drehrad (44) ausgebildet ist oder umgekehrt.

5. Injektor nach Anspruch 3 oder 4, bei dem auf der Rundstange (48) mindestens ein Gleitring (51, 52) angeordnet ist, der in der Führungsbuchse (16) gleitet.

6. Injektor nach einem der Ansprüche 1 bis 5, bei dem die Spindelmutter einteilig mit dem Drehrad (44) oder Grundkörper (6) ausgebildet ist und/oder die Gewindespindel (87) ein mittels eines Befestigungsmittels (92) am Grundkörper (6) oder Drehrad (44) befestigtes Bauteil ist.

7. Injektor nach einem der Ansprüche 1 bis 6, bei dem die Rundstange (48) zugleich als Spindelmutter ausgebildet ist, indem sie mit einem Innengewinde (31, 32) zum Einschrauben der Gewindespindel (87) versehen ist.

8. Injektor nach einem der Ansprüche 1 bis 7, bei dem die Drehentkopplung ein Drehlager (84) ist, das ein erstes und ein zweites Drehlagerelement (85, 86) aufweist, die axial zueinander nicht verlagerbar und relativ zueinander drehbar sind, wobei das erste Drehlagerelement (85) an dem Drehrad (44) und das zweite Drehlagerelement (86) an dem Kolben (70) befestigt ist.

9. Injektor nach Anspruch 8, bei dem das Drehlager ein zum Grundkörper (6) konzentrisches Radiallager ist, vorzugsweise ein radiales Wälzlager (84).

10. Injektor nach einem der Ansprüche 1 bis 9, bei dem der Kolben (70) auf der der ersten Stirnseite (7) zugewandten Seite einen zur Mittelachse (8) des Kolbens (70) konzentrischen, hohlzylindrischen Ringraum (76) aufweist, in dem die Drehentkopplung zumindest teilweise angeordnet ist.

11. Injektor nach einem der Ansprüche 1 bis 10, bei dem der Kolben (70) am Innenumfang mindestens einen ersten Kolbendichtring (72) und am Außenumfang mindestens einen zweiten Kolbendichtring (74) trägt, über die der Kolben (70) an der Innenwand des Zylinderraumes (9) und der Außenwand (11) des Zylinderraumes (9) abgedichtet sind.

12. Injektor nach einem der Ansprüche 1 bis 11, bei dem das Drehrad (44) am äußeren Rand einer kreisscheibenförmigen Deckwand (45) einen umlaufenden, den Außenumfang des Grundkörpers (6) zumindest teilweise überlappenden Mantel (46) aufweist.

13. Injektor nach einem der Ansprüche 1 bis 12, bei dem ein Bauteil, vorzugsweise das Drehrad (44), die Gewindespindel (87) oder der Grundkörper (6) eine Positionsanzeige (94) zum Anzeigen der Stellung des Kolbens (70) im Zylinderraum (9) aufweisen.

14. Injektor nach einem der Ansprüche 1 bis 13, bei dem der Zylinderraum (9) mit einem Belüftungsventil (37) verbunden ist, das eine Schließstellung aufweist, in der es den Zylinderraum (9) von der Umgebung trennt, und eine Öffnungsstellung, in der es den Zylinderraum (9) mit der Umgebung verbindet, sowie eine Betätigungseinrichtung, um das Belüftungsventil (37) von der Schließstellung in die Öffnungsstellung und von der Öffnungsstellung in die Schließstellung zu bringen.

15. Injektor nach einem der Ansprüche 1 bis 14, bei dem an einer zweiten Stirnseite (19) des Grundkörpers (6), die von der ersten Stirnseite (7) abgewandt ist, ein Gerätefuß (95) angeordnet ist, der vorzugsweise eine massive Scheibe und/oder mindestens ein Gummielement und/oder mindestens einen Magneten umfasst oder daraus besteht.

16. Methode zur Zellmanipulation umfassend folgende Schritte:
(a) Auswahl einer zu manipulierenden Zelle;.........................................
(b) Annäherung einer Mikrokapillare, die an den Injektor gemäß einem der Ansprüche 1 -15 angeschlossen ist, an die ausgewählte Zelle;
(c) bei Berührung der Zelle durch die Mikrokapillare, Anlegen eines Unterdrucks vermittelt durch den Injektor zum Halten der Zelle; oder
(d) bei penetrierender Berührung der Zelle durch die Mikrokapillare, Erzeugung eines Überdrucks vermittelt durch den Injektor zum Injizieren einer Lösung, einer Zelle, insbesondere eines Spermiums, und/oder Zellbestandteilen; oder
(e) bei penetrierender Berührung der Zelle durch die Mikrokapillare, Erzeugung eines Unterdrucks vermittelt durch den Injektor, zur Entnahme eines Zellbestandteils, insbesondere eines Zellkerns oder eines Pronukleus.

17. Methode zur Zellmanipulation gemäß Anspruch 16 zur *in-vitro* Fertilisation (IVF), intrazytoplasmatischen Spermieninjektion (ISCI), zum Transfer von Mitochondrien und / oder zum Zellkerntransfer.

18. Verwendung des manuellen Injektors gemäß einem der Ansprüche 1 bis 15 und/ oder der Methode gemäß einem der Ansprüche 16 und 17, in einem medizinischem, biotechnologischen, biochemischen, zellbiologischen und/oder forensischen Labor.

## Claims

1. A manual injector for cell manipulation comprising a displacement device (2) for displacing a fluid, an injection tube (3) connected to the displacement device (2), and a capillary holder (4) connected to the injection tube (3) for holding a microcapillary tube (5) or a microcapillary tube (5) connected to the injection tube (3), wherein the displacement device (2) has the following features:
• a disk-shaped main body (6) that, on a first end face (7), has a hollow cylinder space (9) which is concentric with its middle axis (8),
• a disk-shaped adjusting wheel (44) which is concentric with the main body (6) and is arranged on the first end face (7),
• a helical gear comprising a spindle nut and a threaded spindle (87) engaging therein and arranged on the middle axis (8) of the main body (6), wherein the spindle nut is securely connected to the adjusting wheel (44), and the threaded spindle (87) is securely connected to the main body (6) or vice versa,
• a hollow cylindrical plunger (70) arranged in the cylinder space (9) and sealingly adjacent to the inner perimeter of the inner wall (10) of the cylinder space (9) and the outer perimeter of the outer wall (11) of the cylinder space (9),
• a rotational decoupling that connects the adjusting wheel (44) to the plunger (70) and is designed such that the plunger (70) and adjusting wheel (44) are not displaceable relative to each other in the direction of the middle axis (8) of the main body (6), and are rotatable relative to each other about the middle axis (8), and
• a connection (33) connected to the cylinder space (9) to which the injection tube (3) is connected.

2. The injector according to claim 1, in which a combined linear and pivot bearing (55) is arranged between the main body (6) and adjusting wheel (44) and is designed to guide the adjusting wheel (44) axially in the direction of the middle axis (8) of the main body (6) when displaced relative to the main body (6), and to radially bear the adjusting wheel (44) about the middle axis (8) of the main body (6).

3. The injector according to claim 1 or 2, in which the combined linear and pivot bearing (55) has a hollow cylindrical guide sleeve (16) and a round rod (48) that is guided in the guide sleeve (16) on the middle axis (8) of the main body (6), wherein the guide sleeve (16) is securely connected to the main body (6) and the round rod (48) is securely connected to the adjusting wheel (44), or vice versa.

4. The injector according to claim 3, in which the guide sleeve (16) is designed integrally with the main body (6) and/or the round rod (48) is designed integrally with the adjusting wheel (44), or vice versa.

5. The injector according to claim 3 or 4, in which at least one sliding ring (51, 52) that slides in the guide sleeve (16) is arranged on the round rod (48).

6. The injector according to one of claims 1 to 5, in which the spindle nut is designed integrally with the adjusting wheel (44) or main body (6) and/or the threaded spindle (87) is a component that is fastened to the main body (6) or adjusting wheel (44) by means of a fastening means (92).

7. The injector according to one of claims 1 to 6, in which the round rod (48) is simultaneously designed as a spindle nut in that it is provided with an inner thread (31, 32) for screwing in the threaded spindle (87).

8. The injector according to one of claims 1 to 7, in which the rotational decoupling is a pivot bearing (84) that has a first and a second pivot bearing element (85, 86) that are not axially displaceable relative to each other and are rotatable relative to each other, wherein the first pivot bearing element (85) is fastened to the adjusting wheel (44), and the second pivot bearing element (86) is fastened to the plunger (70).

9. The injector according to claim 8, in which the pivot bearing is a radial bearing that is concentric with the main body (6), preferably a radial roller bearing (84).

10. The injector according to one of claims 1 to 9, in which the plunger (70) has a hollow cylindrical annular space (76) on the side facing the first end face (7) that is concentric with the middle axis (8) of the plunger (70), and in which the rotational decoupling is at least partially arranged.

11. The injector according to one of claims 1 to 10, in which the plunger (70) bears at least one first plunger sealing ring (72) on the inner perimeter and at least one second plunger sealing ring (74) on the outer perimeter by means of which the plunger (70) is sealed against the inner wall of the cylinder space (9) and the outer wall (11) of the cylinder space (9).

12. The injector according to one of claims 1 to 11, in which, on the outer edge of a disk-shaped cover wall (45), the adjusting wheel (44) has a peripheral casing (46) that at least partially overlaps the outer perimeter of the main body (6).

13. The injector according to one of claims 1 to 12, in which a component, preferably the adjusting wheel (44), the threaded spindle (87) or the main body (6), has a position indicator (94) for indicating the position of the plunger (70) in the cylinder space (9).

14. The injector according to one of claims 1 to 13, in which the cylinder space (9) is connected to a ventilation valve (37) that has a closed position in which it separates the cylinder space (9) from the surroundings, and an open position in which it connects the cylinder space (9) to the surroundings, as well as an actuating apparatus in order to bring the ventilation valve (37) from the closed position into the open position, and from the open position into the closed position.

15. The injector according to one of claims 1 to 14, wherein, on a second end face (19) of the main body (6) that faces away from the first end face (7), a device foot (95) is arranged that preferably comprises or consists of a solid disk and/or at least one rubber element and/or at least one magnet.

16. A method for cell manipulation comprising the following steps:
(a) selecting a cell to be manipulated;
(b) bringing a microcapillary tube connected to the injector according to one of claims 1-15 closer to the selected cell;
(c) when the microcapillary tube contacts the cell, applying an underpressure via the injector to hold the cell; or
(d) when the microcapillary tube contacts and penetrates the cell, generating an overpressure via the injector to inject a solution, a cell, in particular a sperm, and/or cell components; or
(e) when the microcapillary tube contacts and penetrates the cell, generating an underpressure via the injector to remove a cell component, in particular a cell nucleus or a pronucleus.

17. The method for cell manipulation according to claim 16 for *in vitro* fertilization (IVF), intra-cytoplasmic sperm injection (ICSI), to transfer mitochondria and/or to transfer cell nuclei.

18. A use of the manual injector according to one of claims 1 to 15 and/or the method according to one of claims 16 and 17, in a medical, biotech, biochemical, cell biology and/or forensic laboratory.

## Revendications

1. Injecteur manuel pour la manipulation de cellules, comportant un dispositif de déplacement (2) pour le déplacement d'un fluide, un tuyau d'injection (3) relié au dispositif de déplacement (2) et un support de capillaire (4) relié au tuyau d'injection (3) pour le support d'un micro-capillaire (5) ou d'un micro-capillaire (5) relié au tuyau d'injection (3), le dispositif de déplacement (2) présentant les caractéristiques suivantes :
• un corps de base (6) en forme de disque circulaire, présentant un espace cylindrique (9) en forme de cylindre creux, concentrique par rapport à son axe médian (8), sur un premier côté frontal (7),
• une roue rotative (44) concentrique par rapport au corps de base (6), en forme de disque circulaire, disposé sur le premier côté frontal (7),
• un engrenage hélicoïdal comportant un écrou de broche et une broche filetée (87) s'engageant dans celui-ci et disposé sur l'axe médian (8) du corps de base (6), l'écrou de broche étant relié fixement à la roue rotative (44) et la broche filetée (87) étant reliée fixement au corps de base (6), ou inversement,
• un piston (70) en forme de cylindre creux disposé dans l'espace cylindrique (9), s'appliquant de façon étanche sur la paroi intérieure (10) de l'espace cylindrique (9) sur le pourtour intérieur ou sur la paroi extérieure (11) de l'espace cylindrique (9) sur le pourtour extérieur,
• un moyen de désaccouplement en rotation reliant la roue rotative (44) au piston (70) et conçu de telle façon que le piston (70) et la roue rotative (44) ne sont pas déplaçables l'un par rapport à l'autre dans la direction de l'axe médian (8) du corps de base (6) et sont rotatifs l'un par rapport à l'autre autour de l'axe médian (8), et
• un raccord (33) relié à l'espace cylindrique (9), auquel est relié le tuyau d'injection (3).

2. Injecteur selon la revendication 1, dans lequel il est prévu, entre le corps de base (6) et la roue rotative (44), un palier linéaire et rotatif combiné (55) conçu pour guider la roue rotative (44) axialement en direction de l'axe médian (8) du corps de base (6) lors d'un déplacement par rapport au corps de base (6), et supporter celle-ci radialement autour de l'axe médian (8) du corps de base (6).

3. Injecteur selon la revendication 1 ou 2, dans lequel le palier linéaire et rotatif combiné (55) présente une douille de guidage (16) en forme de cylindre creux et une barre ronde (48) insérée dans la douille de guidage (16) sur l'axe médian (8) du corps de base (6), la douille de guidage (16) étant reliée fixement au corps de base (6) et la barre ronde (48) étant reliée fixement à la roue rotative (44), ou inversement.

4. Injecteur selon la revendication 3, dans lequel la douille de guidage (16) est formée d'un seul tenant avec le corps de base (6) et/ou la barre ronde (48) est formée d'un seul tenant avec la roue rotative (44), ou inversement.

5. Injecteur selon la revendication 3 ou 4, dans lequel au moins une bague de glissement (51, 52) glissant dans la douille de guidage (16) est disposée sur la barre ronde (48).

6. Injecteur selon l'une des revendications 1 à 5, dans lequel l'écrou de broche est formé d'un seul tenant avec la roue rotative (44) ou le corps de base (6) et/ou la broche filetée (87) est une pièce fixée au corps de base (6) ou à la roue rotative (44) à l'aide d'un moyen de fixation (92).

7. Injecteur selon l'une des revendications 1 à 6, dans lequel la barre ronde (48) est également conçue comme un écrou de broche, étant notamment pourvue d'un filetage intérieur (31, 32) pour le vissage de la broche filetée (87).

8. Injecteur selon l'une des revendications 1 à 7, dans lequel le moyen de désaccouplement en rotation est un palier rotatif (84) présentant un premier et un deuxième élément de palier rotatif (85, 86), lesquels ne sont pas déplaçables l'un par rapport à l'autre et sont rotatifs l'un par rapport à l'autre, le premier élément de palier rotatif (85) étant fixé à la roue rotative (44) et le deuxième élément de palier rotatif (86) étant fixé au piston (70).

9. Injecteur selon la revendication 8, dans lequel le palier rotatif est un palier radial concentrique par rapport au corps de base (6), de préférence un palier à roulement radial (84).

10. Injecteur selon l'une des revendications 1 à 9, dans lequel le piston (70) présente un espace annulaire (76) en forme de cylindre creux, concentrique par rapport à l'axe médian (8) du piston (70), sur le côté tourné vers le premier côté frontal (7), dans lequel le moyen de désaccouplement en rotation est au moins partiellement disposé.

11. Injecteur selon l'une des revendications 1 à 10, dans lequel le piston (70) porte au moins une première bague d'étanchéité de piston (72) sur le pourtour intérieur et au moins une deuxième bague d'étanchéité de piston (74) sur le pourtour extérieur, par le biais desquelles le piston (70) est étanche par rapport à la paroi intérieure de l'espace cylindrique (9) et à la paroi extérieure (11) de l'espace cylindrique (9).

12. Injecteur selon l'une des revendications 1 à 11, dans lequel la roue rotative (44) présente une enveloppe (46) périphérique chevauchant au moins partiellement le pourtour extérieur du corps de base (6), sur le bord extérieur d'une paroi de couverture (45) en forme de disque circulaire.

13. Injecteur selon l'une des revendications 1 à 12, dans lequel une pièce, de préférence la roue rotative (44), la broche filetée (87) ou le corps de base (6), présente un indicateur de position (94) destiné à indiquer la position du piston (70) dans l'espace cylindrique (9).

14. Injecteur selon l'une des revendications 1 à 13, dans lequel l'espace cylindrique (9) est relié à une soupape d'aération (37) présentant une position de fermeture, dans laquelle elle sépare l'espace cylindrique (9) d'avec l'environnement, et une position d'ouverture, dans laquelle elle relie l'espace cylindrique (9) à l'environnement, ainsi qu'un dispositif de commande permettant de faire passer la soupape d'aération (37) de la position de fermeture à la position d'ouverture et de la position d'ouverture à la position de fermeture.

15. Injecteur selon l'une des revendications 1 à 14, dans lequel, sur un deuxième côté frontal (19) du corps de base (6), lequel est opposé au premier côté frontal (7), il est prévu un pied d'appareil (95) comportant de préférence un disque massif et/ou au moins un élément en caoutchouc et/ou au moins un aimant, ou constitué de celui-ci.

16. Procédé de manipulation de cellules, comportant les étapes suivantes :
a) sélection d'une cellule à manipuler ;
b) rapprochement d'un micro-capillaire raccordé à l'injecteur selon l'une des revendications 1 à 15, par rapport à la cellule sélectionnée ;
c) lors d'un contact du micro-capillaire avec la cellule, application d'un vide transmis par l'injecteur pour le maintien de la cellule ; ou
d) lors d'un contact pénétrant du micro-capillaire avec la cellule, génération d'une surpression transmise par l'injecteur pour l'injection d'une solution, d'une cellule, en particulier d'un sperme, et/ou de composants de cellule ; ou
e) lors d'un contact pénétrant du micro-capillaire avec la cellule, génération d'un vide transmis par l'injecteur, pour le retrait d'un composant de cellule, en particulier d'un noyau de cellule ou d'un pronucléus.

17. Procédé de manipulation de cellules selon la revendication 16, pour la fertilisation in vitro (IVF), l'injection de spermes intracytoplasmique (ISCI), le transfert de mitochondries et/ou le transfert de noyaux de cellules.

18. Utilisation de l'injecteur manuel selon l'une des revendications 1 à 15 et/ou du procédé selon l'une des revendications 16 et 17, dans un laboratoire médical, biotechnologique, biochimique, de biologie cellulaire et/ou médico-légal.
